# EUROPEAN PATENT SPECIFICATION

(11) **EP 3 995 077 B1**
(45) Date of publication and mention of the grant of the patent: **04.09.2024**
(21) Application number: 20836514.8
(22) Date of filing: 06.07.2020
(51) Int. Cl.: A61B 5/16, A61B 5/11

(54) **SLEEP-WAKEFULNESS DETERMINATION DEVICE AND PROGRAM**
VORRICHTUNG UND PROGRAMM ZUR BESTIMMUNG DES SCHLAF-WACHZUSTANDS
DISPOSITIF DE DÉTERMINATION DE SOMMEIL/D'ÉTAT DE VEILLE ET PROGRAMME

(30) Priority: 05.07.2019 JP 2019125950
(43) Date of publication of application: 11.05.2022
(73) Proprietor: The University of Tokyo, Bunkyo-ku Tokyo 113-8654 (JP)
(72) Inventor: UEDA, Hiroki, Tokyo 113-8654 (JP); ODE, Koji, Tokyo 113-8654 (JP); SHI, Shoi, Tokyo 113-8654 (JP); MITSUI, Kentaro, Tokyo 113-8654 (JP); KATORI, Machiko, Tokyo 113-8654 (JP)
(74) Representative: Isarpatent
(86) International application number: PCT/JP2020/026345
(87) International publication number: WO 2021/006235

(56) References cited:
- WO-A1-2018/070935
- JP-A- 2006 271 894
- JP-A- 2016 515 463
- JP-A- 2017 169 884
- JP-A- 2017 537 710
- JP-A- 2017 537 731
- JP-A- 2018 520 719
- JP-A- 2019 012 370
- US-A1- 2014 364 770
- US-A1- 2015 190 086
- US-A1- 2016 255 422
- US-A1- 2017 273 617
- COLE,R.J. ET AL.: "Automatic Sleep/Wake Identification From Wrist Activity", SLEEP, vol. 15, no. 5, 1992, pages 461 - 469, XP000856914

## Description

### TECHNICAL FIELD

The present invention relates to a sleep-wakefulness determination apparatus configured to determine the sleep and wakefulness of a user, and a program.

### BACKGROUND ART

It is known that sleep disorders such as insomnia, sleep-disordered breathing, and hypersomnia can be detrimental to health. In order to grasp the sleeping condition of a person, it is necessary to examine the actual condition of how the person actually sleeps, from one night to several days.

The all-night polysomnography (PSG) test, proposed in Patent Document 1 and the like, has been developed as a test to examine a person's sleep state. In PSG, a large number of electrodes and sensors are attached to the body of a subject, and each electrode and sensor is connected to a special measurement device to measure basic data such as electroencephalogram, electrocardiogram, electromyogram, and respiratory status, and then the state of sleep and wakefulness is examined based on the basic data. In Patent Document 2, a device is introduced that recognizes the sleep state of a subject based on the respiratory motion state and the body motion state of the subject, which are determined through a set of time-series measurements performed with a wearable sensor. In Patent Document 3, a machine-learning aided method is introduced to measure the quality of sleep of a subject through sleep staging, which is achieved through the measurement and processing of at least a physiological signal (e.g. an electrocardiogram) obtained with a wireless sensor. In Patent Document 4, a system and method to determine the sleep state of a subject is provided. The determination is performed by processing the data obtained by an accelerometer embedded in a device worn by the user.

### PRIOR ART DOCUMENTS

### Patent Document

[Patent Document 1] Patent Application Publication JP2013-99507
[Patent Document 2] Patent Application Publication US 2017/273617 A1
[Patent Document 3] Patent Application Publication US 2015/190086 A1
[Patent Document 4] Patent Application Publication US 2014/364770 A1

### SUMMARY OF INVENTION

### Problems to be Solved by Invention

A sleep test using PSG, such as that described in Patent Document 1, requires a large number of measurement devices and the location thereof is limited to hospitals and laboratories. This makes it difficult for many people to easily perform the test for longer than a few days. In addition, wearing a lot of electrodes and sensors on the body in an environment different from home causes stress and makes it difficult to sleep. As a result, it is difficult to test the normal sleep state correctly.

In light of the above circumstances, the present invention provides a sleep-wakefulness determination apparatus and program, which determine sleep-wakefulness with a sufficiently high level of accuracy using a small number of wearing devices.

### Means for Solving Problems

According to one aspect of the present invention, there is provided a sleep-wakefulness determination apparatus configured to determine sleep and wakefulness of a user. The present sleep-wakefulness determination apparatus comprises a scalar value calculation unit, a feature value calculation unit, and a sleep-wakefulness determination unit. The scalar calculation unit is configured to calculate scalar value based on each component of the acceleration vector in a part of a body of the user. The feature value calculation unit is configured to calculate feature value for each epoch defined by a predetermined time based on the scalar value. The sleep-wakefulness determination unit is configured to determine the sleep and wakefulness of the user based on the feature value of a desired epoch and the feature value of peripheral epochs included in a plurality of epochs before and after the desired epoch in a time series. The apparatus is characterized in that the scalar value is an L2 norm based on each component of a time difference vector that is a difference vector of two of the acceleration vectors in a time series, the feature value is a histogram generated by dividing the L2 norm or logarithm thereof into classes with a plurality of threshold values, and the predetermined time is set to be equal or less than 5 minutes.

With the sleep-wakefulness determination apparatus, it is possible to determine sleep and wakefulness by the subject to be examined wearing only a bio-acceleration measuring device.

### BRIEF DESCRIPTION OF DRAWINGS

FIG. 1 is a schematic diagram of a sleep-wakefulness determination system for the present system.
FIG. 2A is a hardware configuration of the sleep-wakefulness determination system shown in FIG. 1.
FIG. 2B is a functional block diagram of a controller of the sleep-wakefulness determination system.
FIG. 3 is an example of a wearable device.
FIG. 4 shows how to apply a desired epoch and a plurality of peripheral epochs to a machine learning model.
FIG. 5A is a graph showing L2 norm of acceleration for each epoch.
FIG. 5B is a graph showing L2 norm of time difference of the acceleration for each epoch.
FIG. 5C is a hypnogram of actual sleep.
FIG. 6A is a graph showing the L2 norm of the acceleration per epoch.
FIG. 6B is a hypnogram showing actual sleep.
FIG. 7 is a correct answer percentage and F-value for sleep and wakefulness determination by epoch number and feature value.
FIG. 8 is a flowchart showing a method of determining sleep-wakefulness using the sleep-wakefulness determination system.
FIG. 9A is a hypnogram showing actual sleep.
FIG. 9B is an averaged hypnogram of FIG. 9A.

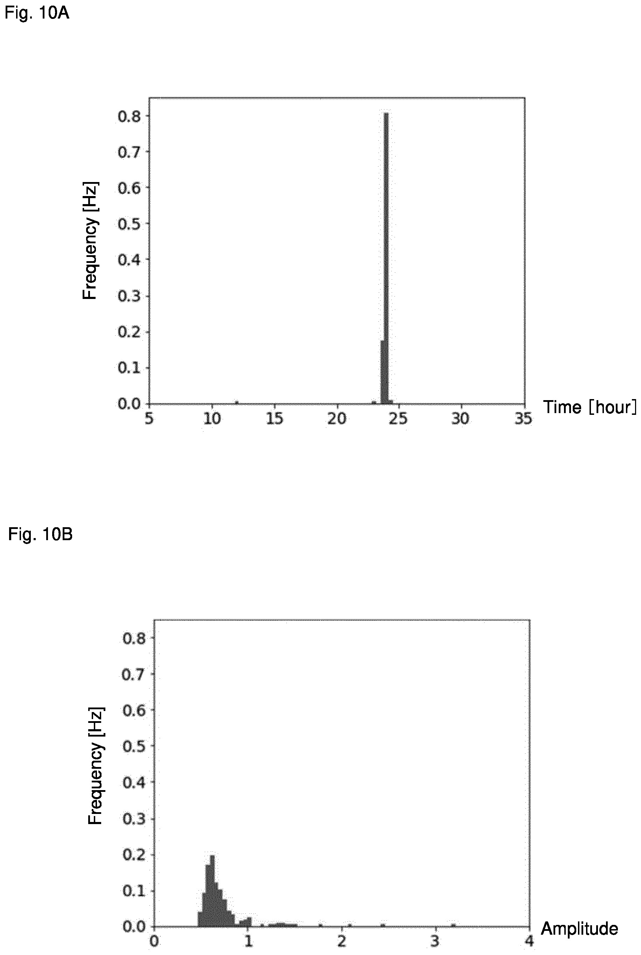

FIG. 10A is a graph showing a period of sleep-wakefulness.
FIG. 10B is an amplitude of the sleep-wakefulness period.
FIG. 11A is a flowchart of a determination result conversion method using the sleep-wakefulness determination system.
FIG. 11B is a flowchart showing a method for converting determination results using the sleep-wakefulness determination system.

### DESCRIPTION OF EMBODIMENTS

Hereinafter, embodiments of the present invention will be described with reference to the drawings. Various features described in the embodiment below can be combined with each other.

A program for realizing a software in the present embodiment may be provided as a non-transitory computer readable medium that can be read by a computer, or may be provided for download from an external server, or may be provided so that the program can be activated on an external computer to realize functions thereof on a client terminal (so-called cloud computing).

In the present embodiment, the "unit" may include, for instance, a combination of hardware resources implemented by circuits in a broad sense and information processing of software that can be concretely realized by these hardware resources. Further, although various information is adopted in the present embodiment, this information can be represented, for example, by physical signal values representing voltage and current, by high and low signal values as a bit set of binary numbers composed of 0 or 1, or by quantum superposition (so-called quantum bit). In this way, communication/operation can be executed on a circuit in a broad sense.

Further, the circuit in a broad sense is a circuit realized by combining at least an appropriate number of a circuit, a circuitry, a processor, a memory, and the like. In other words, it is a circuit includes Application Specific Integrated Circuit (ASIC), Programmable Logic Apparatus (e.g., Simple Programmable Logic Apparatus (SPLD), Complex Programmable Logic Apparatus (CPLD), and Field Programmable Gate Array (FPGA)), and the like.

### 1. Overall configuration

### 1-1. Sleep-wakefulness determination system 1

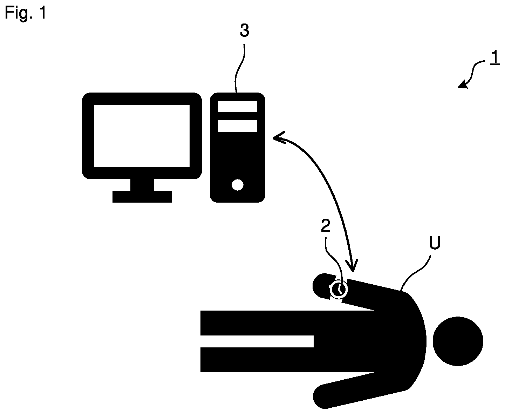

In Section 1, the overall configuration of a sleep-wakefulness determination system 1 will be described. FIG. 1 shows an overview of a configuration of the sleep-wakefulness determination system 1. The sleep-wakefulness determination system 1 is a system comprising a wearable device 2 and a sleep-wakefulness determination apparatus 3, which can exchange information through electrical communication means.

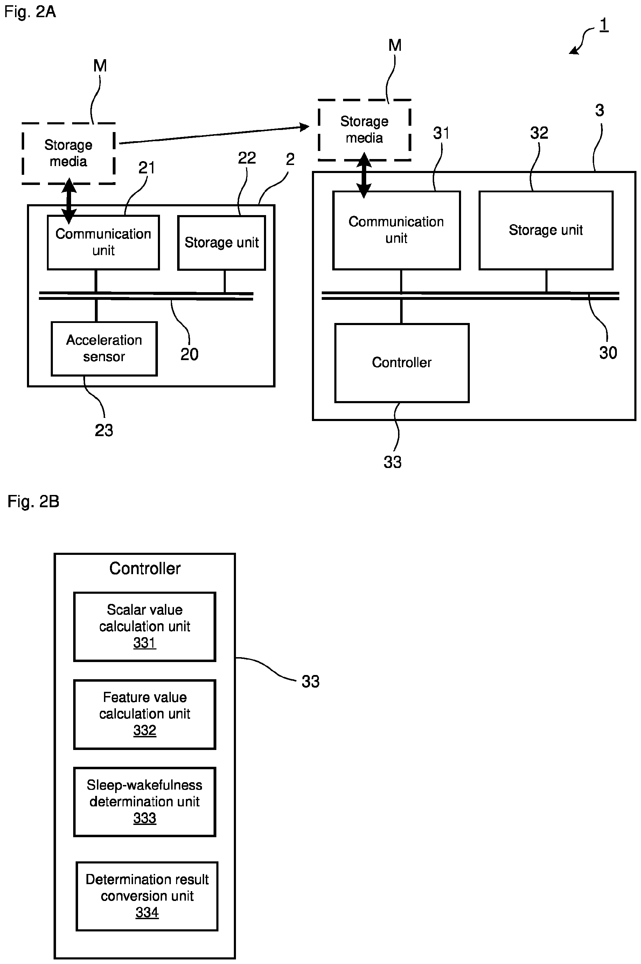

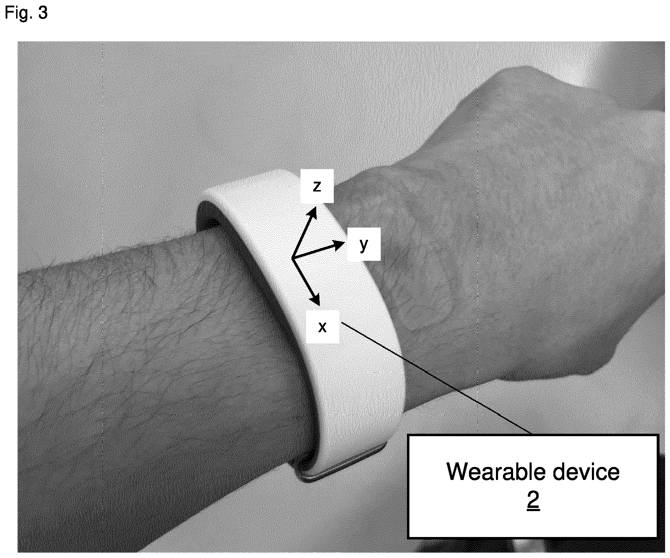

FIG. 2A shows the hardware configuration of the sleep-wakefulness determination system 1 shown in FIG. 1, and FIG. 2B shows the functional block diagram of a controller 33 in the sleep-wakefulness determination apparatus 3. Further, FIG. 3 shows an example of the wearable device 2. Hereinafter, the components of the sleep-wakefulness determination system 1 will be further explained with reference to these figures.

### 1-2. Wearable devices 2

As shown in FIG. 3, the wearable device 2 is a small device that can be worn on the arm of a user U, for example. As shown in FIG. 2A, the wearable device 2 comprises a communication unit 21, a storage unit 22, and an acceleration sensor 23. These components are electrically connected via a communication bus 20 inside the wearable device 2. Each of the components will be described further below.

### (Communication unit 21)

Although wired communication means such as USB, IEEE1394, Thunderbolt, and wired LAN network communication are preferable, the communication unit 21 may include wireless LAN network communication, mobile communication such as 3G/LTE/5G, Bluetooth (registered trademark) communication or the like as necessary. In particular, in the present embodiment, the communication section 21 is preferably configured to write information including time-series three-dimensional (3-D) acceleration vectors v(x, y, z) measured by the acceleration sensor 23 described below to external storage media M. The type and form of the storage media M are not particularly limited, and for example, flash memory, card-type memory, optical disk, etc. may be employed as appropriate.

### (Storage unit 22)

The storage unit 22 stores various information defined by the aforementioned description. The storage unit 22 can be implemented, for instance, as a storage device such as a solid state drive (SSD), or as a memory such as a random access memory (RAM) that stores temporarily necessary information (argument, array, or the like) regarding program operation, or any combination thereof. In particular, the storage unit 22 can store information including time-series 3-D acceleration vectors v(x, y, z) measured by the acceleration sensor 23 described below. It may be implemented to store the information directly in the aforementioned storage media M without going through the storage unit 22.

### (Acceleration sensor 23)

The acceleration sensor 23 is configured to measure the acceleration of a part of a body (e.g., an arm) of the user U as 3-D vector information. In other words, information including time-series 3-D acceleration vectors v(x, y, z) can be acquired from the user U.

### 1-3. Sleep-wakefulness determination apparatus 3

As shown in FIG. 2A, the sleep-wakefulness determination apparatus 3 comprises a communication unit 31, a storage unit 32, and a controller 33, and these components are electrically connected via a communication bus 30 inside the sleep-wakefulness determination apparatus 3. Each of the components will be described further below.

### (Communication unit 31)

Although wired communication means such as USB, IEEE1394, Thunderbolt, and wired LAN network communication are preferable, the communication unit 21 may include wireless LAN network communication, mobile communication such as 3G/LTE/5G, Bluetooth (registered trademark) communication or the like as necessary. In particular, in the present embodiment, it is preferable to implement the communication unit 31 as a storage media reading unit configured to read information stored in external storage media M. The storage media M stores information including time-series 3-D acceleration vectors v(x, y, z) acquired from the user U by the wearable device 2. As a result, the communication unit 31, which is a storage media reading unit, can read the 3-D acceleration vector v(x, y, z) stored in the storage media M.

### (Storage unit 32)

The storage unit 32 stores various information defined by the aforementioned description. The storage unit 32 can be implemented, for instance, as a storage device such as a solid state drive (SSD), or as a memory such as a random access memory (RAM) that stores temporarily necessary information (argument, array, or the like) regarding program operation, or any combination thereof.

In particular, the storage unit 32 stores a scalar value calculation program for calculating a scalar value a based on each component (x, y, z) of the 3-D acceleration vector v(x, y, z) at a part of the body of the user U. The storage unit 32 also stores a feature value calculation program for calculating a feature value f(N) for each epoch defined by a predetermined time based on the scalar value a. Further, the storage unit 32 stores a sleep-wakefulness determination program for determining the sleep and wakefulness of the user U based on the feature value f(N) of the desired epoch and the feature value f(N±δ) of peripheral epochs included in the plurality of epochs before and after the desired epoch in a time series. Further, the storage unit 32 stores various programs with respect to the sleep-wakefulness determination apparatus 3 executed by the controller 33, etc. in addition to the above.

Further, the storage unit 32 stores a machine learning model allowed to learn correlation of the feature value f(N) of the desired epoch, the feature value f(N±δ) of the peripheral epoch and the sleep and wakefulness of the user U. Preferably, conventional algorithms can be employed for the algorithm for such machine learning as appropriate. For example, logistic regression, random forest, XGBoost, multilayer perceptron (MLP), or the like can be adopted. In addition, each time the sleep-wakefulness determination apparatus 3 is used, machine learning using the results thereof as training data can be further performed to update such machine learning model.

### (Controller 33)

The controller 33 processes and controls overall operation regarding the sleep-wakefulness determination apparatus 3. The controller 33 is implemented as, for instance, an unshown central processing unit (CPU). The controller 33 realizes various functions with respect to the sleep-wakefulness determination apparatus 3 by reading out a predetermined program stored in the storage unit 32. Specifically, the scalar value calculation function for calculating a scalar value a based on each component (x, y, z) of the 3-D acceleration vector v(x, y, z) in a part of the body of the user U, the feature value calculation function for calculating the feature value f(N) for each epoch defined by a predetermined time based on the scalar value a, the sleep and wakefulness determination function for determining the sleep and wakefulness of the user U based on the feature value f(N) of the desired epoch and the feature value f(N±δ) of peripheral epochs included in the plurality of epochs before and after the desired epoch in a time series, in such epochs or the like are included.

In other words, the information processing by software (stored in the storage unit 32) is specifically realized by hardware (controller 33), in such a manner that the controller 33 may be executed as a scalar value calculation unit 331, a feature value calculation unit 332, and a sleep-wakefulness determination unit 333 as shown in FIG. 2B. In FIGs. 2A and 2B, although it is described as a single controller 33, it is not limited thereto, and may be implemented with a plurality of controllers 33 for each function. Further, combination thereof may also be implemented. Hereinafter, the scalar value calculation unit 331, the feature value calculation unit 332, and the sleep-wakefulness determination unit 33 will be further described in detail.

### (Scalar value calculation unit 331)

The scalar value calculation unit 331 is configured to execute the information processing by software (stored in the storage unit 32) specifically realized by hardware (controller 33). The scalar value calculation unit 331 calculates a scalar value a based on each component (x, y, z) of the 3-D acceleration vector v(x, y, z) at a part of the body of the user U. The scalar value a is the L2 norm (so-called magnitude) of v(x, y, z). Of course, the scalar value a can also be the L1 norm.

Further, the scalar value calculation unit 331 calculates the scalar value a (the L2 norm) based on each component of the time difference vector Δv(x, y, z) acquired from the 3-D acceleration vector v(x, y, z). Here, the time difference vector Δv(x, y, z) is a difference vector between two 3-D acceleration vectors v_1(x, y, z) and v_2 (x, y, z) in a time series. The two 3D acceleration vectors v(x, y, z) at adjacent times are more preferably employed. Due to this process, the effect of gravitational acceleration can be suppressed compared to the case in which the 3-D acceleration vector v(x, y, z) is used directly.

Alternatively, the scalar value calculation unit 331 calculates the scalar value a (the L2 norm) based on each component of the n-th-order time derivative vector v^n(x, y, z) acquired from the 3-D acceleration vector v(x, y, z). Here, the n-th-order time derivative vector v^n(x, y, z) is the n-th-order time derivative of the 3-D acceleration vector where n is a natural number (n≥1). Due to this process, the effect of gravitational acceleration can be suppressed compared to the case in which the 3-D acceleration vector v(x, y, z) is used directly.

The values of n are, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, and may be in the range between any two of the numbers indicated above.

### (Feature value calculation Unit 332)

The feature value calculation unit 332 is configured to execute the information processing by software (stored in the storage unit 32) specifically realized by hardware (controller 33). The feature value calculation unit 332 calculates the feature value f(N) for each epoch specified by the predetermined time based on the scalar value a calculated by the scalar value calculation unit 331. These will be described in detail in Section 2.

### (Sleep-wakefulness determination unit 333)

The sleep-wakefulness determination unit 333 is configured to execute the information processing by software (stored in the storage unit 32) specifically realized by hardware (controller 33). The sleep-wakefulness determination unit 333 determines the sleep and wakefulness of the user U based on the feature value f(N) of the desired epoch and the feature value f(N±δ) of the peripheral epochs included in the plurality of epochs before and after the desired epoch in a time series, in the epochs. At this time, the sleep-wakefulness determination unit 333 can determine such sleep and wakefulness based on the above-described machine learning model stored in the storage unit 32.

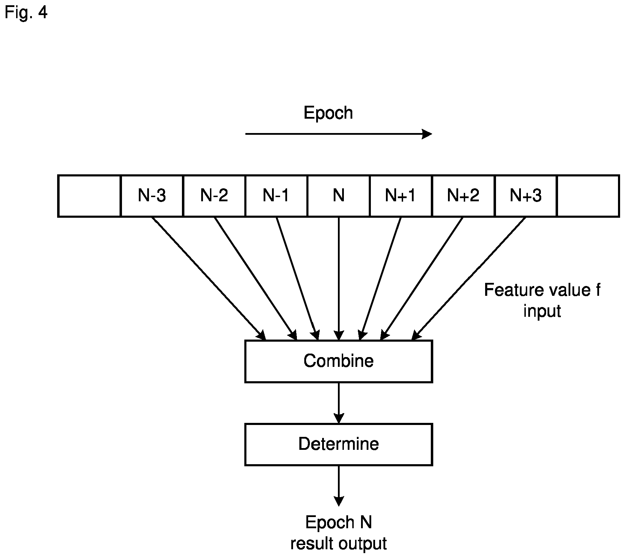

FIG. 4 shows how the desired epoch and multiple peripheral epochs are applied to the machine learning model. The n-th epoch is the desired epoch, and ±3th epochs before and after the desired epoch are selected as peripheral epochs. In other words, using the feature value f(N) of the desired epoch and the feature value f(N±1, 2, 3) of the peripheral epochs included in the third before and after the desired epoch in a time series as input, the sleep-wakefulness determination unit 333 determines the sleep and wakefulness of the user U based on the aforementioned machine learning model stored in the storage unit 32. The number of epochs is, of course, only an example and is not limited thereto.

That is, the number of epochs can be, for example, 1, 2, 3, 4, 5, 6, 7, 8, 9, 10, 11, 12, 13, 14, 15, 16, 17, 18, 19, 20, 21, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, and may be in the range between any two of the numbers indicated above.

### (Determination result conversion unit 334)

The determination result conversion unit 334 is configured to execute the information processing by software (stored in the storage unit 32) specifically realized by hardware (controller 33). It is preferable that the controller 33 further comprises the determination result conversion unit 334. The determination result conversion unit 334 converts the results determined by the sleep-wakefulness determination unit 333. These will be described in detail in Section 4.

### 2. Details

Section 2 describes the details of the sleep-wakefulness determination system 1 with reference to the experimental data. In the experiment, the predetermined time to define the epoch was set to 30 seconds, but it should be noted that this is not the limit of the experiment.

### 2-1. Correlation between acceleration vector and sleep

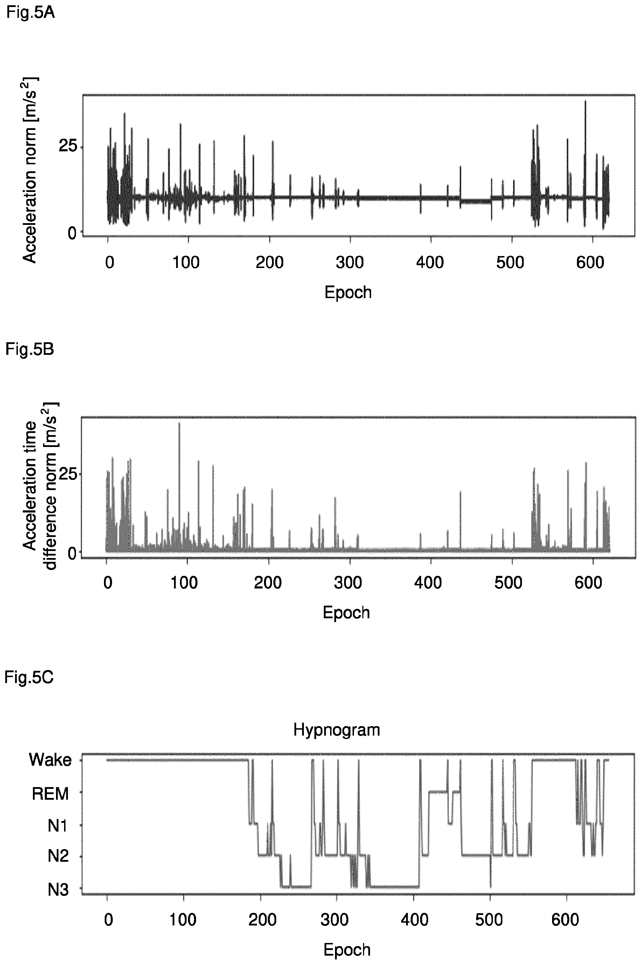

FIG. 5a shows the L2 norm of the 3D acceleration vector v(x, y, z) per epoch. FIG. 5b shows the L2 norm of the time difference vector Δv(x, y, z) for each epoch. FIG. 5C is a hypnogram showing actual sleep. It is confirmed that the user U is awake at the timing when the numerical value of the L2 norm of the 3-D acceleration vector v(x, y, z) or the time difference vector Δv(x, y, z) is large. In other words, the correlation between the acceleration at a part of the body of user U acquired by the wearable device 2 and the sleep and wakefulness of the user U was confirmed. This is the basic principle of the sleep-wakefulness determination system 1 of the present embodiment.

### 2-2. Feature Value

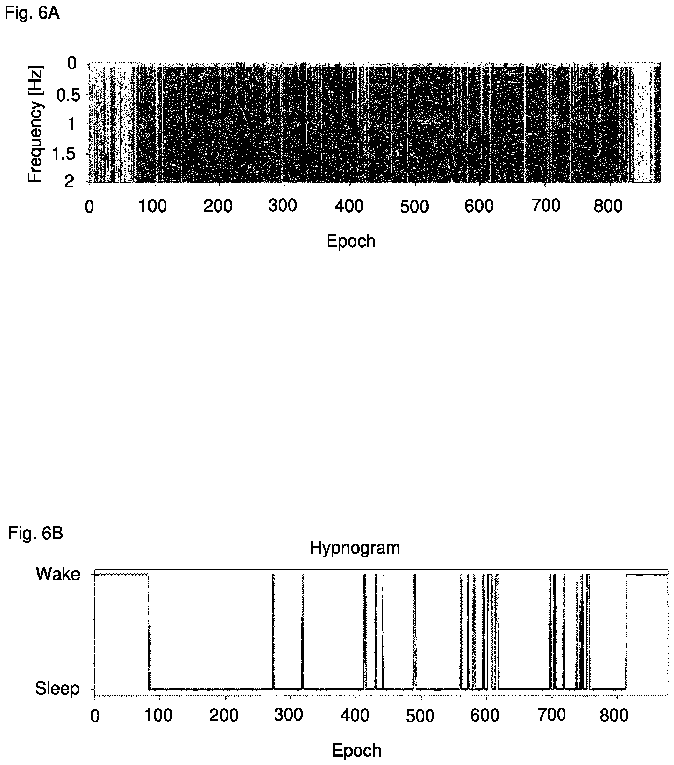

As mentioned above, the present sleep-wakefulness determination system 1 extracts the feature value f(N) from the 3-D acceleration vector v (x, y, z) etc. from the L2 norm, and uses the feature value f(N) to determine sleep and wakefulness. Specifically, the feature value f(N) is a histogram generated by dividing the scalar value a or the logarithm thereof into classes with multiple thresholds, or a power spectrum based on the product of the scalar value a multiplied by a window function. For example, FIG. 6a shows the logarithmic power spectrum of the time difference vector Δv(x, y, z). The hypnogram correlating to the logarithmic power spectrum is also shown in FIG. 6B.

### 2-3. Correct answer percentage for the number of epochs

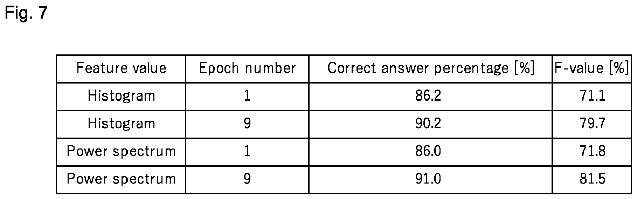

As described above, the sleep-wakefulness determination unit 333 determines the sleep and wakefulness of the user U based on the feature value f(N) of the desired epoch and the feature value f(N±δ) of the peripheral epochs included in the plurality of epochs before and after the desired epoch in a time series, in the epochs. The number of epochs (the sum of one desired epoch and the peripheral epochs) should be selected appropriately. For reference, a comparison between a case where the number of epochs is 1 and a case where the number of epochs is 9 is shown in FIG. 7. In both the histogram and the power spectrum, it is confirmed that the correct answer percentage and the F-value (ratio of standard deviation) in the case of 9 are increased compared to the case of 1.

### 3. Sleep-wakefulness determination method

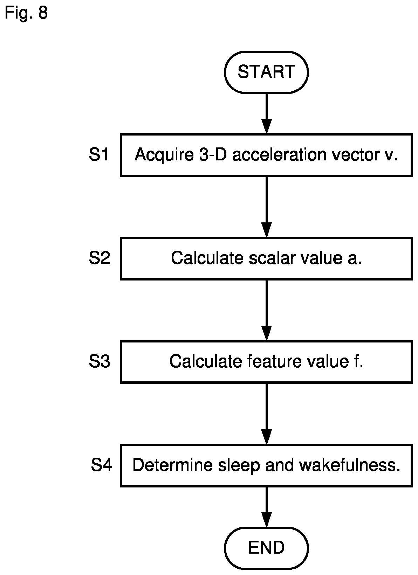

In Section 3, a method of determining the sleep-wakefulness using the sleep-wakefulness determination system 1 is described according to a flowchart shown in FIG. 8.

### [START]

### (Step S1)

Using the wearable device 2 worn by the user U, information including the time-series 3D acceleration vector v(x, y, z) at a part of the body of the user U is acquired. The information acquired thereby is read into the sleep-wakefulness determination apparatus 3 via the storage media M.

### (Step S2)

Following the step S1, the scalar value calculation unit 331 in the sleep-wakefulness determination apparatus 3 calculates the scalar value a based on each component (x, y, z) of the 3-D acceleration vector v(x, y, z) at a part of the body of the user U.

### (Step S3)

Following the step S2, the feature value calculation unit 332 in the sleep-wakefulness determination apparatus 3 calculates the feature value f(N) for each epoch defined by a predetermined time based on the scalar value a calculated by the scalar value calculation unit 331.

### (Step S4)

Following the step S3, the sleep-wakefulness determination unit 333 in the sleep-wakefulness determination device 3 determines the sleep and wakefulness of the user U by employing the feature value f(N) of the desired epoch and the feature value f(N±δ) of the peripheral epochs included in a plurality of epochs before and after the desired epoch in a time series, in the epochs, as inputs to the machine learning model stored in the storage unit 32

### [END]

Due to the present method of determining sleep and wakefulness, the sleep and wakefulness with a sufficiently high degree of accuracy using only a few devices can be determined.

### 4. Determination result conversion method

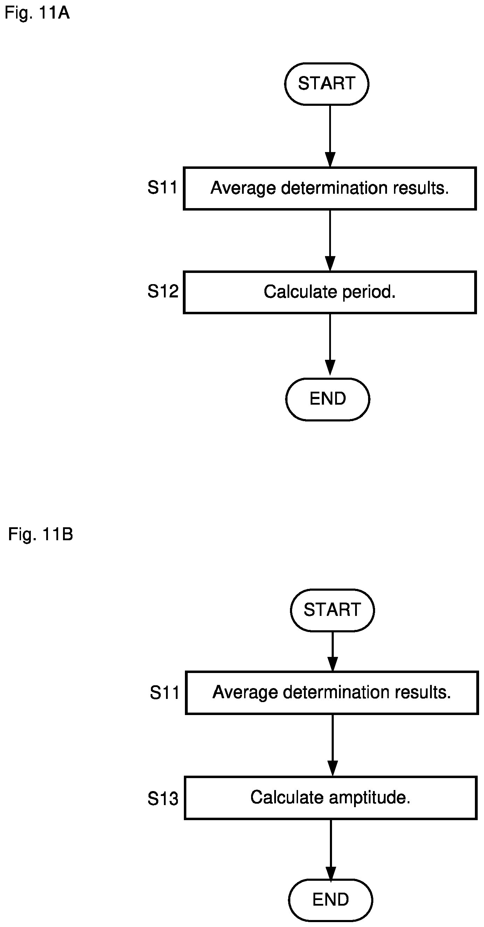

In Section 4, a method of converting the results of sleep-wakefulness determination using the sleep-wakefulness determination system 1 is explained according to flowcharts shown in FIG. 11A and 11B.

### [START]

### (Step S11)

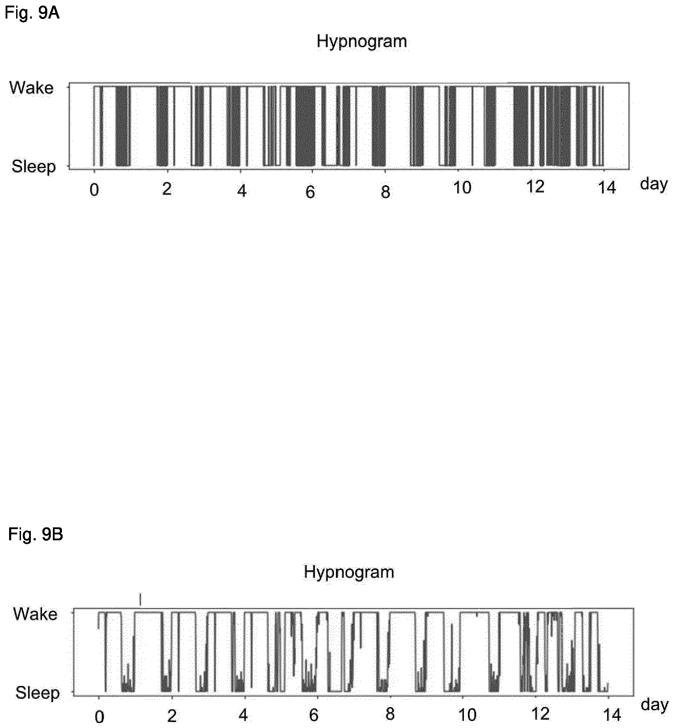

In the result determined by the sleep-wakefulness determination unit 333, as shown in Fig. 9A, the result is set to 0 or 1 (sleep or wakefulness) every 30 seconds. In this step, as shown in FIG. 9B, the determination results are averaged every 10 minutes, and the data is converted smoothly into data having a value between 0 and 1 with 0.05 increments. The specified time (10 minutes) for averaging the determination results and the increment width (0.05) of the averaged results are both examples and can be changed as necessary.

### (Step S12)

Following the step S11, the period of the sleep-wakefulness is calculated using the Chi-square periodogram method.

### (Step S13)

Following the step S11, the coefficient of variation (standard deviation divided by the mean) is calculated to determine the amplitude of sleep-wakefulness.

### [END]

According to the method of converting the determination results, the sleep and wakefulness of the user U can be examined from various perspectives.

### 5. Modification

In Section 5, variations of the sleep-wakefulness determination system 1 according to the present embodiment will be described. That is, the sleep-wakefulness determination system 1 according to the present embodiment may be further devised in the following manner.

First, instead of the storage media M, the communication unit 21 in the wearable device 2 may transmit information including a time-series 3-D acceleration vector v(x, y, z) in a part of the body of the user U to the communication unit 31 in the sleep-wakefulness determination apparatus 31, via wireless communication. In other words, the communication unit 31 is configured to communicate with the wearable device 2 (including the acceleration sensor 23) worn by the user U on a part of the body thereof so as to receive the 3-D acceleration vector v(x, y, z) measured by the acceleration sensor 23.

Secondly, the wearable device 2 and the sleep-wakefulness determination apparatus 3 may be configured as a single unit. In other words, the sleep-wakefulness determination apparatus 3 may be a wearable device 2 to be worn by the user U on a part of the body, and may further comprise an acceleration sensor 23. The acceleration sensor 23 may be configured to measure a 3-D acceleration vector v(x, y, z).

### 6. Conclusion

As described above, the present embodiment makes it possible to implement a sleep-wakefulness determination apparatus 3 configured to determine sleep and wakefulness with a sufficiently high degree of accuracy using a small number of wearing devices.

There is provided a sleep-wakefulness determination apparatus configured to determine sleep and wakefulness of a user, comprising a scalar calculation unit configured to calculate a scalar value based on each component of an acceleration vector in a part of a body of the user; a feature value calculation unit configured to calculate a feature value for each epoch defined by a predetermined time based on the scalar value; and a sleep-wakefulness determination unit configured to determine the sleep and wakefulness of the user based on the feature value of a desired epoch and the feature value of peripheral epochs included in a plurality of epochs before and after the desired epoch in a time series, in the epoch.

Software for implementing the sleep-wakefulness determination apparatus 3 as hardware so as to determine the sleep and wakefulness with sufficiently high accuracy using a small number of wearing devices can also be implemented as a program. Such a program may be provided as a non-transitory computer readable medium that can be read by a computer, or may be provided for download from an external server, or may be provided so that the program can be activated on an external computer to realize functions thereof on a client terminal (so-called cloud computing).

There is provided a program which allows a computer to function as a sleep-wakefulness determination apparatus configured to determine sleep and wakefulness of a user, the apparatus comprising: a scalar calculation unit configured to calculate a scalar value based on each component of an acceleration vector in a part of a body of the user; a feature value calculation unit configured to calculate a feature value for each epoch defined by a predetermined time based on the scalar value; and a sleep-wakefulness determination unit configured to determine the sleep and wakefulness of the user based on the feature value of a desired epoch and the feature value of peripheral epochs included in a plurality of epochs before and after the desired epoch in a time series.

Finally, various embodiments of the present invention have been described, but these are presented as examples and are not intended to limit the scope of the invention.

### REFERENCE SIGNS LIST

1: Sleep-wakefulness determination system
2: Wearable device
20: Communication bus
21: Communication unit
22: Storage unit
23: Acceleration sensor
3: Sleep-wakefulness determination apparatus
30: Communication bus
31: Communication unit
32: Storage unit
33: Controller
331: Scalar value calculation unit
332: Feature value calculation unit
333: Sleep-wakefulness determination unit
334: Determination result conversion unit
M: Storage media
U: User
a: Scalar value
f: Feature value
v: 3-D acceleration vector
v^n: n-th-order time derivative vector
Δv: Time difference vector

## Claims

1. A sleep-wakefulness determination apparatus (3) configured to determine sleep and wakefulness of a user (U), comprising:
a scalar value calculation unit (331) configured to calculate a scalar value (a) based on each component of an acceleration vector in a part of a body of the user (U);
a feature value calculation unit (332) configured to calculate a feature value for each epoch defined by a predetermined time based on the scalar value (a); and
a sleep-wakefulness determination unit (333) configured to determine the sleep and wakefulness of the user (U) based on the feature value of a desired epoch and the feature value of peripheral epochs included in a plurality of epochs before and after the desired epoch in a time series, in the epoch,
**characterized in that**:
the scalar value (a) is an L2 norm based on each component of a time difference vector that is a difference vector of two of the acceleration vectors in a time series,
the feature value is a histogram generated by dividing the L2 norm or logarithm thereof into classes with a plurality of threshold values, and
the predetermined time is set to be equal or less than 5 minutes.

2. The sleep-wakefulness determination apparatus (3) according to claim 1, wherein the scalar value calculation unit (331) is configured to calculate the scalar value (a) based on each component of an n-th-order time derivative vector, the n-th-order time derivative vector being a vector in which the acceleration vector is differentiated by n-th-order time derivative, and n being a natural number.

3. The sleep-wakefulness determination apparatus (3) according to any one of claims 1 to 2, wherein
the feature value is a power spectrum based on a product of the scalar value (a) multiplied by a window function.

4. The sleep-wakefulness determination apparatus (3) according to any one of claims 1 to 3, further comprising a storage unit (32) which stores a machine learning model allowed to learn correlation of the feature value of the desired epoch, the feature value of the peripheral epochs and the sleep and wakefulness of the user (U), wherein
the sleep-wakefulness determination unit (333) is configured to determine the sleep and wakefulness based on the machine learning model.

5. The sleep-wakefulness determination apparatus (3) according to any one of claims 1 to 4, further comprising a storage media reading unit configured to read the acceleration vector stored in storage media.

6. The sleep-wakefulness determination apparatus (3) according to any one of claims 1 to 4, further comprising a communication unit (31) configured
to communicate with an acceleration sensor (23) worn on a part of a body of the user (U), and
to receive the acceleration vector measured by the acceleration sensor (23).

7. The sleep-wakefulness determination apparatus (3) according to any one of claims 1 to 4, the apparatus being a wearable device (2) worn on a part of a body of the user (U), and further comprising an acceleration sensor (23) configured to measure the acceleration vector.

8. The sleep-wakefulness determination apparatus (3) according to any one of claims 1 to 7, further comprising a determination result conversion unit (334) configured to convert a result determined by the sleep-wakefulness determination unit (333).

9. A program which allows a computer to function as a sleep-wakefulness determination apparatus (3) configured to determine sleep and wakefulness of a user (U), the apparatus comprising:
a scalar value calculation unit (331) configured to calculate a scalar value (a) based on each component of an acceleration vector in a part of a body of the user (U);
a feature value calculation unit (332) configured to calculate a feature value for each epoch defined by a predetermined time based on the scalar value (a); and
a sleep-wakefulness determination unit (333) configured to determine the sleep and wakefulness of the user (U) based on the feature value of a desired epoch and the feature value of peripheral epochs included in a plurality of epochs before and after the desired epoch in a time series, in the epoch, **characterized in that**
the scalar value (a) is an L2 norm based on each component of a time difference vector that is a difference vector of two of the acceleration vectors in a time series, wherein
the feature value is a histogram generated by dividing the L2 norm or logarithm thereof into classes with a plurality of threshold values, and wherein
the predetermined time is set to be equal or less than 5 minutes.

## Patentansprüche

1. Vorrichtung zur Bestimmung des Schlaf-Wach-Zustands (3), dafür eingerichtet, den Schlaf- und Wach-Zustand eines Benutzers (U) zu bestimmen, und umfassend:
eine Skalarwertberechnungseinheit (331), die dafür eingerichtet ist, einen Skalarwert (a) auf der Grundlage jeder Komponente eines Beschleunigungsvektors in einem Teil eines Körpers des Benutzers (U) zu berechnen;
eine Merkmalswertberechnungseinheit (332), die dafür eingerichtet ist, einen Merkmalswert für jede durch eine zuvor festgelegte Zeit definierte Epoche auf der Grundlage des Skalarwertes (a) zu berechnen; und
eine Einheit zur Bestimmung des Schlaf-Wach-Zustands (333), die dafür eingerichtet ist, den Schlaf- und Wach-Zustand des Benutzers (U) auf der Grundlage des Merkmalswertes einer gewünschten Epoche und des Merkmalswertes von Randepochen, die in mehreren Epochen vor und nach der gewünschten Epoche in einer Zeitreihe enthalten sind, in der Epoche zu bestimmen,
**dadurch gekennzeichnet, dass**:
der Skalarwert (a) eine L2-Norm ist, die auf jeder Komponente eines Zeitdifferenzvektors basiert, der ein Differenzvektor von zwei Beschleunigungsvektoren in einer Zeitreihe ist,
der Merkmalswert ein Histogramm ist, das durch Unterteilen der L2-Norm oder ihres Logarithmus in Klassen mit mehreren Schwellenwerten generiert wird, und
die zuvor festgelegte Zeit auf maximal 5 Minuten eingestellt ist.

2. Vorrichtung zur Bestimmung des Schlaf-Wach-Zustands (3) nach Anspruch 1, wobei
die Skalarwertberechnungseinheit (331) dafür eingerichtet ist, den Skalarwert (a) auf der Grundlage jeder Komponente eines Zeitableitungsvektors n-ter Ordnung zu berechnen, wobei der Zeitableitungsvektor n-ter Ordnung ein Vektor ist, bei dem der Beschleunigungsvektor durch eine Zeitableitung n-ter Ordnung differenziert wird und n eine natürliche Zahl ist.

3. Vorrichtung zur Bestimmung des Schlaf-Wach-Zustands (3) nach einem der Ansprüche 1 und 2, wobei
der Merkmalswert ein Leistungsspektrum ist, das auf einem Produkt des mit einer Fensterfunktion multiplizierten Skalarwertes (a) beruht.

4. Vorrichtung zur Bestimmung des Schlaf-Wach-Zustands (3) nach einem der Ansprüche 1 bis 3, umfassend des Weiteren eine Speichereinheit (32), die ein Maschinenlernmodell speichert, dem es erlaubt ist, eine Korrelation zwischen dem Merkmalswert der gewünschten Epoche, dem Merkmalswert der Randepochen und dem Schlaf- und Wach-Zustand des Benutzers (U) zu erlernen, wobei
die Vorrichtung zur Bestimmung des Schlaf-Wach-Zustands (333) dafür eingerichtet ist, den Schlaf- und Wach-Zustand auf der Grundlage des Maschinenlernmodells zu bestimmen.

5. Vorrichtung zur Bestimmung des Schlaf-Wach-Zustands (3) nach einem der Ansprüche 1 bis 4, umfassend des Weiteren eine Speichermedienleseeinheit, die dafür eingerichtet ist, den in Speichermedien gespeicherten Beschleunigungsvektor zu lesen.

6. Vorrichtung zur Bestimmung des Schlaf-Wach-Zustands (3) nach einem der Ansprüche 1 bis 4, umfassend des Weiteren eine Kommunikationseinheit (31), die dafür eingerichtet ist,
mit einem Beschleunigungssensor (23) zu kommunizieren, der an einem Teil eines Körpers des Benutzers (U) getragen wird, und
den durch den Beschleunigungssensor (23) gemessenen Beschleunigungsvektor zu empfangen.

7. Vorrichtung zur Bestimmung des Schlaf-Wach-Zustands (3) nach einem der Ansprüche 1 bis 4, wobei die Vorrichtung eine am Körper tragbare Vorrichtung (2) ist, die an einem Teil eines Körpers des Benutzers (U) getragen wird, und des Weiteren einen Beschleunigungssensor (23) umfasst, der zum Messen des Beschleunigungsvektors eingerichtet ist.

8. Vorrichtung zur Bestimmung des Schlaf-Wach-Zustands (3) nach einem der Ansprüche 1 bis 7, umfassend des Weiteren eine Bestimmungsergebnisumwandlungseinheit (334), die dafür eingerichtet ist, ein durch die Schlaf-Wach-Bestimmungseinheit (333) bestimmtes Ergebnis umzuwandeln.

9. Programm, das es einem Computer erlaubt, als eine Vorrichtung zur Bestimmung des Schlaf-Wach-Zustands (3) zu fungieren, die dafür eingerichtet ist, den Schlaf- und Wach-Zustand eines Benutzers (U) zu bestimmen, wobei die Vorrichtung umfasst:
eine Skalarwertberechnungseinheit (331), die dafür eingerichtet ist, einen Skalarwert (a) auf der Grundlage jeder Komponente eines Beschleunigungsvektors in einem Teil eines Körpers des Benutzers (U) zu berechnen;
eine Merkmalswertberechnungseinheit (332), die dafür eingerichtet ist, einen Merkmalswert für jede durch eine zuvor festgelegte Zeit definierte Epoche auf der Grundlage des Skalarwertes (a) zu berechnen; und
eine Einheit zur Bestimmung des Schlaf-Wach-Zustands (333), die dafür eingerichtet ist, den Schlaf- und Wach-Zustand des Benutzers (U) auf der Grundlage des Merkmalswertes einer gewünschten Epoche und des Merkmalswertes von Randepochen, die in mehreren Epochen vor und nach der gewünschten Epoche in einer Zeitreihe enthalten sind, in der Epoche zu bestimmen,
**dadurch gekennzeichnet, dass**
der Skalarwert (a) eine L2-Norm ist, die auf jeder Komponente eines Zeitdifferenzvektors basiert, der ein Differenzvektor von zwei Beschleunigungsvektoren in einer Zeitreihe ist, wobei
der Merkmalswert ein Histogramm ist, das durch Unterteilen der L2-Norm oder ihres Logarithmus in Klassen mit mehreren Schwellenwerten generiert wird, und wobei
die zuvor festgelegte Zeit auf maximal 5 Minuten eingestellt ist.

## Revendications

1. Appareil de détermination du sommeil et de l'éveil (3) configuré pour déterminer le sommeil et l'éveil d'un utilisateur (U), comprenant :
une unité de calcul de valeur scalaire (331) configurée pour calculer une valeur scalaire (a) sur la base de chaque composante d'un vecteur d'accélération dans une partie du corps de l'utilisateur (U) ;
une unité de calcul de valeur caractéristique (332) configurée pour calculer une valeur caractéristique pour chaque époque définie par un temps prédéterminé sur la base de la valeur scalaire (a) ; et
une unité de détermination du sommeil et de l'éveil (333) configurée pour déterminer le sommeil et l'éveil de l'utilisateur (U) sur la base de la valeur caractéristique d'une époque souhaitée et de la valeur caractéristique d'époques périphériques incluses dans une pluralité d'époques avant et après l'époque souhaitée dans une série temporelle, dans l'époque,
**caractérisé en ce que** :
la valeur scalaire (a) est une norme L2 basée sur chaque composante d'un vecteur de différence temporelle qui est un vecteur de différence de deux des vecteurs d'accélération dans une série temporelle,
la valeur caractéristique est un histogramme généré en divisant la norme L2 ou son logarithme en classes avec une pluralité de valeurs seuils, et
le temps prédéterminé est fixé à une durée égale ou inférieure à 5 minutes.

2. Appareil de détermination du sommeil et de l'éveil (3) selon la revendication 1, dans lequel
l'unité de calcul de valeur scalaire (331) est configurée pour calculer la valeur scalaire (a) sur la base de chaque composante d'un vecteur de dérivée temporelle d'ordre n, le vecteur de dérivée temporelle d'ordre n étant un vecteur dans lequel le vecteur d'accélération est différencié par une dérivée temporelle d'ordre n, et n étant un nombre naturel.

3. Appareil de détermination du sommeil et de l'éveil (3) selon l'une quelconque des revendications 1 à 2, dans lequel
la valeur caractéristique est un spectre de puissance basé sur un produit de la valeur scalaire (a) multipliée par une fonction de fenêtre.

4. Appareil de détermination du sommeil et de l'éveil (3) selon l'une quelconque des revendications 1 à 3, comprenant en outre une unité de stockage (32) qui stocke un modèle d'apprentissage automatique pouvant apprendre une corrélation entre la valeur caractéristique de l'époque souhaitée, la valeur caractéristique des époques périphériques et le sommeil et l'éveil de l'utilisateur (U), dans lequel
l'unité de détermination du sommeil et de l'éveil (333) est configurée pour déterminer le sommeil et l'éveil sur la base du modèle d'apprentissage automatique.

5. Appareil de détermination du sommeil et de l'éveil (3) selon l'une quelconque des revendications 1 à 4, comprenant en outre une unité de lecture du support de stockage configurée pour lire le vecteur d'accélération stocké sur le support de stockage.

6. Appareil de détermination du sommeil et de l'éveil (3) selon l'une quelconque des revendications 1 à 4, comprenant en outre une unité de communication (31) configurée pour communiquer avec un capteur d'accélération (23) porté sur une partie du corps de l'utilisateur (U), et
recevoir le vecteur d'accélération mesuré par le capteur d'accélération (23).

7. Appareil de détermination du sommeil et de l'éveil (3) selon l'une quelconque des revendications 1 à 4, l'appareil étant un dispositif portable (2) porté sur une partie du corps de l'utilisateur (U), et comprenant en outre un capteur d'accélération (23) configuré pour mesurer le vecteur d'accélération.

8. Appareil de détermination du sommeil et de l'éveil (3) selon l'une quelconque des revendications 1 à 7, comprenant en outre une unité de conversion de résultat de détermination (334) configurée pour convertir un résultat déterminé par l'unité de détermination du sommeil et de l'éveil (333).

9. Programme permettant à un ordinateur de fonctionner comme un appareil de détermination du sommeil et de l'éveil (3) configuré pour déterminer le sommeil et l'éveil d'un utilisateur (U), l'appareil comprenant :
une unité de calcul de valeur scalaire (331) configurée pour calculer une valeur scalaire (a) sur la base de chaque composante d'un vecteur d'accélération dans une partie du corps de l'utilisateur (U) ;
une unité de calcul de valeur caractéristique (332) configurée pour calculer une valeur caractéristique pour chaque époque définie par un temps prédéterminé sur la base de la valeur scalaire (a) ; et
une unité de détermination du sommeil et de l'éveil (333) configurée pour déterminer le sommeil et l'éveil de l'utilisateur (U) sur la base de la valeur caractéristique d'une époque souhaitée et de la valeur caractéristique d'époques périphériques incluses dans une pluralité d'époques avant et après l'époque souhaitée dans une série temporelle, dans l'époque,
**caractérisé en ce que**
la valeur scalaire (a) est une norme L2 basée sur chaque composante d'un vecteur de différence temporelle qui est un vecteur de différence de deux des vecteurs d'accélération dans une série temporelle, dans lequel la valeur caractéristique est un histogramme généré en divisant la norme L2 ou son logarithme en classes avec une pluralité de valeurs seuils, et dans lequel le temps prédéterminé est fixé à une durée égale ou inférieure à 5 minutes.
